# EUROPEAN PATENT APPLICATION

(11) **EP 1 897 585 A1**
(43) Date of publication of application: **12.03.2008**
(21) Application number: 07017434.7
(22) Date of filing: 06.09.2007
(51) Int. Cl.: A61M 39/24, A61M 39/26, F16K 7/14, F16K 15/14, F16K 15/18, A61M 5/168, A61M 39/22

(54) **Anti-siphon control valve**

(30) Priority: 07.09.2006 US 824783 P
(71) Applicant: Barak, Swi, Caesarea 38900 (IL)
(72) Inventor: Barak, Swi, Caesarea 38900 (IL)
(74) Representative: Kohler Schmid Möbus

(57) **Abstract**

An anti-siphon control valve including a housing fitted with a first port inflow, being in fluid communication with a first chamber, and a second port outflow, being in fluid communication with a second chamber coaxial with the first chamber. A fluid flow path extends between the chambers. A resilient membrane is tensioned over the fluid flow path and over chamber so as to close the fluid flow path. The membrane has one face exposed to atmospheric pressure. The arrangement is such that when differential pressure across the membrane exceeds predetermined pressure threshold the membrane flexes and opens the fluid path allowing fluid flow between the first chamber and the second chamber.

## Description

### FIELD OF THE INVENTION

The present invention is in the field of fluid flow control valves, such as for use in medical administration sets etc.

### BACKGROUND OF THE INVENTION

It is often required to provide an anti-siphon control valve which operates within a specific pressure range. An example for such an instance is a set for administrating medical liquids to a patient, in which a container holding the liquid is supported by a stand at a height of typically about two meters or at times being provided within a pressure device.

It is required to ensure that liquid contained within the container freely flows in a direction towards the patient and to prevent flow in an opposite direction, i.e. in a direction from the patient.

A variety of liquid flow valves are known. Typical prior art flow valves are fitted on a fluid supply line and comprise a membrane sealingly engaging a closure between an upstream portion and a downstream portion of the supply line, which upon exposure to a predetermined pressure, the membrane deforms to open a flow path towards the upstream portion.

By another example, the membrane comprises one or more apertures such as pre-pierced or pre-slotted apertures which in a non-deformed position, the apertures are closed. However, upon deformation of the membrane under a predetermined pressure, the apertures open and liquid flow is enabled through the supply line.

Typically anti-siphon flow control valves are unidirectional, i.e. permitting flow from an inlet port towards an outlet port but not *vice versa.* In cases where the valve is Bi-directional, then the pressure required to initiate flow is typically identical in both directions of operation.

Other types of flow valves are of complex structure and are provided with mechanical components such as springs, gaskets and pistons and thus render the valve complexity and of essentially high costs.

Many of the therefore known anti-siphon control valves are not provided with priming means and where such means are provided, there are no arrangements for retaining the priming means in their priming position, but rather it is required to manually keep the priming means depressed.

It is an object of the present invention to provide an improved fluid flow anti-siphon control valve in which the above referred to drawbacks are essentially reduced or overcome.

### SUMMARY OF THE INVENTION

According to a preferred aspect of the present invention there is provided an anti-siphon fluid valve comprising a housing fitted with a first port being in flow communication with a first chamber, and a second port being in fluid communication with a second chamber coaxial with the first chamber; a fluid flow path extending between the chambers; a resilient membrane tensioned over the fluid flow path and over the second chamber so as to close the fluid flow path, said membrane having one face thereof exposed to atmospheric pressure; the arrangement being such that when differential pressure across the membrane exceeds a predetermined pressure threshold the membrane flexes and opens the fluid flow path allowing fluid flow between the first chamber and the second chamber.

According to a preferred embodiment of the present invention, the valve is formed with a priming member for manually displacing the membrane to disengage from the fluid flow path and open it. Preferable, the priming member is a stem projecting from a wall of the first chamber connected to other portions of the first chamber via a flexible zone, whereby displacing the wall towards the membrane entails displacing the membrane and priming the valve.

Still preferably, the priming member is biased to disengage from the membrane. By one of its designs, the flexible zone of the first chamber is resilient and is biased to resume its original shape.

By still a preferred embodiment of the present invention, the valve further comprises a retention member for retaining the priming member in its displaced position, thereby keeping the fluid flow path open. By one application of this preferred embodiment, there is further provided a locking arrangement for arresting the retention member in a position in which the fluid flow path is kept open.

Desirably, the first port and the second port coaxially extend from the housing along an axis normal to the axis of the chambers, and the retention member is a bridge-like element comprising a retaining portion for engaging the wall portion, and two arms, each engaged with one of the first or second port, respectively, and being rotatable about the port's axis.

By a preferred design of the valve in accordance with the present invention, the first chamber is a well-like enclosure having a bottom wall portion communicating with other portions of the first chamber via a flexible zone. A top, rimmed edge separates between the first and second chambers and forms the fluid flow path and over which the membrane is tensioned.

Still preferably, the housing further comprises a venting cover positioned in close proximity to the flexible membrane whereby in its flexed state the membrane bears against the venting cover. The venting cover is fitted with one or more venting apertures. Among its tasks, the venting cover also secures the membrane to the housing along a peripheral edge thereof

In order to avoid blocking of the one or more venting apertures upon flexing of the membrane, the one or more apertures are preferably formed at peripheral portions of the venting cover.

It will be appreciated that the valve according to the present invention may be Bi-directional valve and the cross-sectional area of the membrane over the first chamber may be similar or different to the cross-sectional area of the membrane over the second chamber, whereby the differential pressure required to open the flow path in one direction may be different than the differential pressure required to open the flow path in the other direction.

### BRIEF DESCRIPTION OF THE DRAWING

In order to understand the invention and to see how it may be carried out in practice, a preferred embodiment will now be described, by way of a non-limiting example only, with reference to the accompanying drawings in which:
Fig. 1. is a longitudinal cross-section of the valve according to the present invention, the valve is in its closed position.
Fig. 2. is a longitudinal cross-section of the valve according to the present invention, the valve is in its open position, with a priming retainer in its locked position;

### DETAILED DESCRIPTION OF A SPECIFIC EMBODIMENT

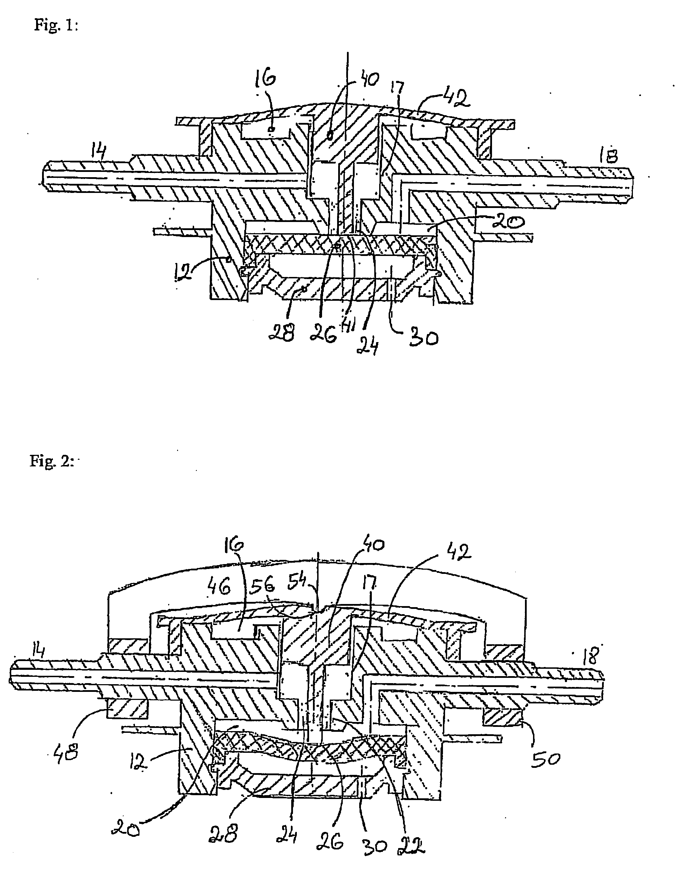

Attention is directed to Figs. 1 and 2 of the drawings illustrating a valve generally designated 10 comprising a cylindrical housing 12 formed with a first; inlet port 14 extending into a fast inlet chamber 16, the latter being coaxial with the housing 12. First chamber 16 is formed by a well-like member 17 snapingly engaging the housing 12.

A second, outlet port 18 is coaxial with the inlet port 14 and extends into a second, outlet chamber 20, which in fact is in the shape of an annulus which is coaxial with the first chamber 16 and which have a common separating wall 22 terminating at a rimmed annular edge 24 which defines a fluid flow path between the first and second chambers as will hereinafter be explained.

A resilient membrane 26 is securely attached to the housing 12 by a venting cover 28 snapingly fitted to the housing 12, where the membrane 26 sealingly bears against the rimmed annular edge 24, thus sealing the first and second chambers 16 and 20 respectively, and the flow path 24 extending between the chambers.

As can be seen in Fig. 1, the venting cover 28 is fitted with a venting aperture 30 at a peripheral portion of the cover, for the reason to become apparent hereinafter. It will further be noted that the vented cover 28 is in close proximity to the membrane 26, though leaving a clearance 32 therebetween, wherein atmospheric pressure exists.

A stem-like priming member 40 extends from a flexible bottom wall portion 42 of the first chamber 16, said stem-member 40 extending almost towards a bottom surface of the membrane 26 leaving a clearance between its upper end 41 and membrane 26.

A retention member 46 has a bridge-like shape with two annular portions 48 and 50 rotatably engaged over inlet port 14 and outlet port 18, respectively. A bridging portion 46 has a central portion thereof a buldge 54 for engagement with depression 56 formed at a lowermost, central portion of the well-like portion 17.

Further attention is now directed to Fig. 2 of the drawings in which the anti-siphon valve is shown in its locked and open position after applying pressurized fluids through inlet port 14 entailing flexion of the membrane 26 to disengage the rimmed annular edge 24, thus opening the fluid flow path between the first, inlet chamber 16 and the second, outlet chamber 20, permitting fluid egress via outlet port 18, as illustrated by arrows.

It will be noted that in the open position of the valve, as seen in Fig. 2, the membrane 26 bears against a central portion of the venting cover 28 while the venting aperture 30 remains unblocked, thus ensuring that the space 32 remains at atmospheric pressure.

A person versed in the art will have no difficulty in realizing that the first and second ports and the first and second chambers may change their functions, respectively, i.e. the second port 18 may serve as an inlet port and the first inlet port 14 may serve as an outlet port with the second chamber 20 serving as an inlet chamber and the first chamber 16 serving as an outlet chamber, respectively. It will further be appreciated by a skilled person that changing the cross-sectional area of the diaphragm 26 over the first chamber 16 or over the second chamber 20 will determine the differential pressure required for opening the valve. Accordingly, the valve may be used as a uni-directional valve or as a Bi-directional valve with equal or different pressures required for opening the valve in either direction.

Attention is now directed to Fig. 2 of the drawings. In certain instances, it is required to prime a valve, e.g. to drain gases from a liquid supply line or to rinse a supply line prior to administrating fluids. A non-limiting example, where such priming means are required are valves used in medical liquid administration.

When it is required to momentarily prime the valve, the bottom portion 42 of well-like member 17 of the first chamber 16 is depressed upwards against the resiliency of the resilient annular segment 42, whereby the top end 41 of priming member 40 encounters the membrane 26 and deforms it to the position seen in Fig. 2, i.e., opening the flow path 24 between the chambers, enabling flow through the valve.

However, when it is required to retain the valve 10 in its primed position, i.e., when the pressure through the inlet port does not reach the predetermined pressure threshold, then it is possible to open the valve and keep it in its open position by swinging the retention member 46 into the position seen in Fig. 2 in which the well-like member 17 of the first chamber 16 is depressed, thus entailing deformation of membrane 26 into its open position. In order to ensure that the valve remains in its open position, bulge 54 snaps into depression 56 thus ensuring that the retention member 46 does not swing out of engagement with the bottom portion of the well-like member 17.

It will be appreciated to a person versed in the art that the design of the housing may be different than the specific design illustrated herein, e.g. the manner in which the venting cover and the bottom portion are engaged with the housing, location of resilient portion, etc., mutatis mutandis.

## Claims

1. An Anti-Siphon control valve (10) comprising a housing (12) fitted with a first port (14) inflow, being in fluid communication with a first chamber (16), and a second port (18) outflow, being in fluid communication with a second chamber (20) coaxial with the first chamber (16); a fluid flow path (24) extending between the chambers (16; 20); a resilient membrane (26) is tensioned over the fluid flow path (24) and over chamber (20) so as to close the fluid flow path (24), said membrane (26) having one face thereof exposed to atmospheric pressure; the arrangement being such that when differential pressure across the membrane (26) exceeds predetermined pressure threshold the membrane flexes and opens the fluid path (24) allowing fluid flow between the first chamber (16) and the second chamber (20).

2. An Anti-Siphon control valve (10) with orifice (24) and second flow chamber (20) having different areas in contact with the flexible membrane (26) establish in effect a Bi-directional valve that shall open in one direction with excessive pressure difference, hence turning the valve into a Uni-directional valve.

3. An Anti-Siphon fluid control valve according to Claim 1, wherein the valve (10), further comprises a priming member (40) for manually displacing the membrane (26) to disengage from the fluid flow path (24).

4. An Anti-Siphon fluid control valve according to Claim 3, wherein the priming member is a stem (40) projecting from a wall portion (42) of the first chamber (16) whereby displacing the wall towards the membrane entails priming of the valve (10).

5. An Anti-Siphon fluid control valve according to Claim 4, wherein the wall portion (42) is flexible and connected to other wall portions of the first chamber (16).

6. An Anti-Siphon fluid control valve according to Claim 4, wherein the priming member (40) is biased to disengage the membrane (26).

7. An Anti-Siphon fluid control valve according to Claim 5, wherein the flexible wall (42) is biased to resume its original shape.

8. An Anti-Siphon fluid control valve according to Claim 3, further comprising a retention member (46) for retaining the priming member (40) in its displaced position thereby keeping the fluid flow path (24) open.

9. An Anti-Siphon fluid control valve according to Claim 8, comprising a locking arrangement (54; 56) for arresting the retention member (46) in a position in which the fluid path (24) is kept open.

10. An Anti-Siphon fluid control valve according to Claim 8, wherein the first port (14) and the second port (18) co-axially extend from the housing (12) along an axis normal to the axis of the chambers, and where the retention member (46) is a bridge-like element having a retaining portion (54) for engaging the priming member, and two arms (48; 50) each engaged with one of the first or second ports (14; 18) and being rotatable about the ports' axis.

11. An Anti-Siphon fluid control valve according to Claim 1, wherein the first chamber (16) is a well-like enclosure having a flexible deformable zone at a bottom wall and a top rimmed edge (22) forming the fluid flow path (24) and over which the membrane (26) is tensioned.

12. An Anti-Siphon fluid control valve according to Claim 1, wherein the area difference between orifice (24), in communication with the surface of membrane (26), and the area of the second chamber (20), in communication with the surface of membrane (26), entail different pressures required to deform the membrane (26), and thus constitute in effect a uni-directional valve, having a fluid flow from inflow port (14) towards outflow port (18).

13. An Anti-Siphon fluid control valve according to Claim 1, wherein the housing further comprises a venting cover (28) positioned in close proximity to the flexible membrane (26) whereby in its flexed state the membrane (26) bears against the venting cover (28), the venting cover (28) fitted with one or more venting apertures (30).

14. The pattern of the cross-section of membrane (26) shall be U-shaped such that it caps the venting cover (28) with an exact fit

15. An Anti-Siphon fluid control valve according to Claim 12, wherein the one or more apertures (30) are formed at peripheral portions of the venting cover (28).

16. An Anti-Siphon fluid control valve according to Claim 1, wherein the second chamber (20) is in the shape of an annulus detached from the first chamber (16).

17. A system comprising of a Peristaltic pump and an administration set, baring the valve (10) that will predetect the event of a user forgetting the lever (46) connected at (54). Detection should occur automatically before pump starts operation based on the pressure (lack of pressure) the valve mounted below the pump will initiate.
